# EUROPEAN PATENT APPLICATION

(11) **EP 1 736 168 A1**
(43) Date of publication of application: **27.12.2006**
(21) Application number: 05720572.6
(22) Date of filing: 11.03.2005
(51) Int. Cl.: A61K 38/00, A61K 45/00, A61K 48/00, A61P 9/00, A61P 9/02, A61P 11/00, G01N 33/15, G01N 33/50, C12N 15/09, A01K 67/027

(54) **PROTEASE INHIBITOR AND PREVENTIVES OR REMEDIES FOR DISEASES**

(30) Priority: 11.03.2004 JP 2004069835; 29.03.2004 JP 2004094065
(71) Applicant: KURUME UNIVERSITY, Kurume-shi, Fukuoka 830-0011 (JP)
(72) Inventor: HOSHINO, Tomoaki, c/o Kurume University, Kurume-shi Fukuoka, 8300011 (JP); AIZAWA, Hisamichi, c/o Kurume University, Kurume-shi Fukuoka, 8300011 (JP)
(74) Representative: Copp, David Christopher
(86) International application number: PCT/JP2005/004301
(87) International publication number: WO 2005/087249

(57) **Abstract**

[Problems]

To provide a novel protease inhibitor and curative remedies and a therapeutic method for chronic obstructive pulmonary disease, immunodeficiency syndrome, alveolar proteinosis and circulatory diseases.

[Means for solving problems]

A protease inhibitor and a preventive or a remedy for chronic obstructive pulmonary disease or immunodeficiency syndrome **characterized by** containing at least one member selected from among redox active proteins and genes encoding the same and a preventive or a remedy for chronic obstructive pulmonary disease, alveolar proteinosis or circulatory diseases **characterized by** containing at least one member selected from IL-18 and a gene encoding the same.

## Description

### BACKGROUND OF THE INVENTION

### Cross reference to related applications

This is a national phase of International Patent Application No. PCT/JP2005/004301, with an international filing date of March 11, 2005. This application based upon and claims the benefit of priority from the prior Japanese Patent Applications No. 2004-069835, filed March 11, 2004 and No. 2004-094065, filed March 29, 2004, the entire contents of which are incorporated herein by reference.

### Technical Field

The present invention relates to an inhibitor of proteases that are one of the causes for chronic obstructive pulmonary disease and to preventive or therapeutic agents for chronic obstructive pulmonary disease, immunodeficiency syndrome, pulmonary proteinosis, and cardiovascular disease.

### Description of the Related Art

Chronic Obstructive Pulmonary Disease (referred to below as COPD) is a disease accompanied by progressive obstructive ventilatory impairment due to emphysema, to chronic bronchitis, or to the combination of these. Airflow limitation observed in COPD is caused by increased airway resistance due to a disorder in peripheral airways and by decreased elasticity and contractility in lungs due to emphysema. These causes incur the airflow in different level for each case of COPD. For most COPD patients, emphysema is a more prominent cause than the disorder in peripheral airways. Many epidemiologic researches have shown that the largest risk factor for emphysema is smoking. National Heart, Lung and Blood Institution (NHLBI) and World Health Organization (WHO) jointly published Global Initiative for Chronic Obstructive Lung Disease (GOLD) in the year of 2001. The report showed that world average morbidity of COPD is 9.34/1000 for male and 7.33/1000 for female, which is one of highest rate among other diseases according to a research in 1990. In the United States, respiratory insufficiency due to COPD is now the fourth most cause of death. In Japan, death caused by COPD has increased four times during the last 30 years, according to a report by Ministry of Health, Labour and Welfare, Japan. Conventional therapeutic agents for COPD consist of bronchodilator or steroid, or of the combination of these. These agents, however, are not effective enough and therefore new therapeutic agents are needed. As another therapeutic agent for COPD, tiotropium bromide hydrate is available, but still more effective agent is needed.

A therapeutic agents and a therapy for complete cure of COPD has not been established so far.

The following references are incorporated herein by reference: 1. Pauwels, R. A., Buist, A. S., Calverley, P. M., Jenkins, C. R., and Hurd, S. S. "Global strategy for the diagnosis, management, and prevention of chronic obstructive pulmonary disease. NHLBI/WHO Global Initiative for Chronic Obstructive Lung Disease (GOLD) Workshop summary." (American journal of respiratory and critical care medicine, vol.163: PP.1256-1276, 2001.) 2. Barnes, P. J. "Novel approaches and targets for treatment of chronic obstructive pulmonary disease." (American journal of respiratory and critical care medicine, vol.160: S72-79, 1999.)

Pulmonary alveolar proteinosis is a disease that causes surfactant protein and phospholipid to accumulate in the alveolus lumen. Recent research showed that autoantibody which inhibits the activity of GM-CSF (Granulocyte-Macrophage Colony-Stimulating Factor) was found in 90 % cases of pulmonary alveolar proteinosis. However, any therapy for complete cure of COPD has not been known. Current therapy for COPD consists of cleansing pulmonary alveoli with physiological saline using a bronchoscope.

COPD causes some related cardiovascular diseases including circulatory failure, such as a cardiac failure (e.g. cor pulmonale caused by load on right heart observed in chronic pulmonary diseases including COPD), and subsequent pulmonary insufficiency and pulmonary hypertension.

Accordingly, there is a need for a therapeutic agent and a therapy for complete cure of COPD, pulmonary alveolar proteinosis, circulatory failure (e.g. pulmonary insufficiency, cardiac failure, and pulmonary hypertension).

In 1980s, AIDS (acquired immune deficiency syndrome) was a disease of high death rate. AIDS is caused by HIV (Human Immunnodeficiency Virus) that infects CD4 (a kind of antigenic protein that consists of a single chain transmembrane glycoprotein with a molecular weight of 59kDa) positive cells to destroy the immune system. The patients gradually lose their nature until they die.

Recently, however, all HIV-infected people in USA or other countries around the world do not necessarily follow such disease course. In XI International Conference on AIDS held in Vancouver, Canada in July 1996, many encouraging reports for concerned parties were made. For example, David Ho from Aaron Diamond AIDS Research Center of New York reported that a combination of a protease inhibitor and an AZT (zidovudine; a kind of reverse transcriptase inhibitor)-type antiviral drug, which has been available since 1991, effectively prevents AIDS. Using several agents for inhibiting HIV increase in human bodies to prevent AIDS is known as cocktail therapy.

It was reported that the cocktail therapy helps increasing immunocytes in AIDS patients and to reduce the HIV virus in blood below the detection limit, although it cannot cure AIDS completely.

Protease inhibitors are known to act the most effectively when administered with the conventional reverse transcriptase inhibitors, such as AZT, d4T (stavudine), ddI (didanosine). Such treatment is known as HAART therapy (Highly Active Antiretroviral Therapy). As a matter of fact, between the years 1996 to 1998, death because of HIV infection decreased by more than 70 % in the USA so that AIDS became no longer among ten most frequent causes of death. In 1998, fatality rate of AIDS recorded the lowest number since the start of the survey in 1987 and is expected to decrease further.

In December 1995, FDA (Food and Drug Administration) approved the first protease inhibitor "saquinavir". By the spring of 1996, FDA approved other two protease inhibitors, "ritonavir" and "indinavir". HIV protease inhibitors have superior inhibitory action but they have also many adverse effects.

It is known that thioredoxin is markedly expressed in the serum of HIV patients (Proc Natl Acad Sci USA. 2001 Feb 27;98(5):2688-93). The reason for such marked expression has not been revealed.

Elastase is known as a protease that hydrolyzes elastin, which is a main component of elastic fiber of the lung. It is known that elastase induces pulmonary emphysema when intratracheally administered. Elastase-administered animals are used as animal models of pulmonary emphysema.

### SUMMARY OF THE INVENTION

Considering that elastase induces pulmonary emphysema, it was assumed that inhibiting proteases, such as elastase, helps for therapy of pulmonary emphysema among other diseases included in COPD. Based on this assumption, protease inhibitors were searched, and then protein with redox activity was found. It was found that protein redox activity intensively inhibited COPD. The protein with redox activity was also expected to be used in an AIDS therapy as a protease inhibitor used solely or in combination with other drugs for a cocktail therapy (e.g. HAART therapy). Further, since the protein with redox activity has a kind of IL-18 inhibiting activity, the present inventors deduced that other IL-18 inhibitors can be used as therapeutic agents for COPD. Therefore, one object of the present invention is to provide protease (elastase) inhibitors, therapeutic agents for COPD, AIDS, pulmonary alveolar proteinosis, cardiac failure, hepatic insufficiency, and cardiovascular diseases (e.g. circulatory failure accompanied by pulmonary hypertension).

The present invention provides the followings:
[1] protease inhibitors comprising at least one selected from (1) to (4) below.
[2] the protease inhibitors wherein the protease is selected from metalloprotease, serin protease, and cysteine protease
[3] preventive or therapeutic agents for chronic obstructive pulmonary disease or AIDS comprising at least one selected from (1) to (4) below
[4] preventive or therapeutic agents for chronic obstructive pulmonary diseases comprising at least one selected from (5) to (8) below
[5] preventive or therapeutic agents for pulmonary alveolar proteinosis comprising at least one selected form (5) to (8) below
[6] preventive or therapeutic agents for cardiovascular diseases comprising at least one selected from (5) to (8) below.

(1) redox activity protein
(2) protein with a similar activity to the redox activity protein, comprising an amino acid sequence in which one or several amino acids are deleted from, replaced with, or added to the redox activity protein
(3) genes that encode (1)
(4) genes that encode (2)
(5) interleukin-18 inhibitor
(6) protein with an activity of inhibiting interleukin-18, comprising an amino acid sequence in which one or several amino acids are deleted from, replaced with, or added to the interleukin-18 inhibitor
(7) genes that encode (1)
(8) genes that encode (2)

The protease inhibitor that comprises protein with redox activity or the gene that encodes such protein, and the preventive or therapeutic agent for COPD according to the present invention not only intensively inhibits COPD but also can be used in an AIDS therapy as a protease inhibitor used solely or in combination with other drugs for a cocktail therapy (e.g. HAART therapy). The protein with redox activity or the gene encoding such protein originally exists in cells and has less adverse effects than those conventional protease inhibitors used in cocktail therapies for AIDS. The preventive or therapeutic agents that comprises IL-18 inhibitors or the gene encoding the IL-18 effectively cures COPD, AIDS, pulmonary alveolar proteinosis, cardiac failure, hepatic insufficiency, and cardiovascular diseases (e.g. circulatory failure accompanied by pulmonary hypertension).

### BRIEF DESCRIPTION OF THE DRAWINGS

FIG. 1 shows a micrographic image of the lung tissue of group 1 (control mice) of example 2 (HE staining, × 40 magnification).
FIG. 2 shows a micrographic image of the lung tissue of group 2 (pathologic mouse models 1) of example 2 (HE staining, × 40 magnification).

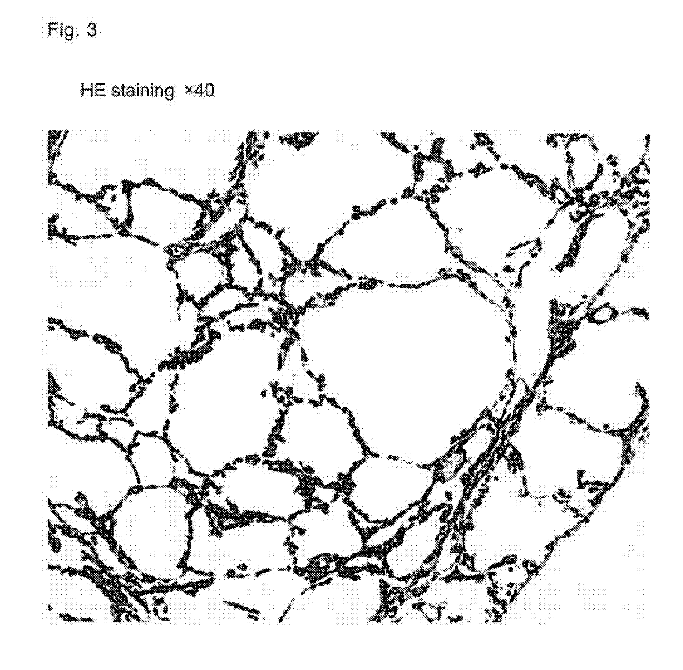

FIG. 3 shows a micrographic image of the lung tissue of group 3 (pathologic mouse models 2) of example 2 (HE staining, × 40 magnification).
FIG. 4 shows a micrographic image of the lung tissue of group 4 (predisposing factor and therapeutic agent administered mice) of example 2 (HE staining, × 40 magnification).
FIG. 5 shows the average length of pulmonary alveoli (mean linear intercept: Lm) of group 1 to group 4 in example 2.
FIG. 6 shows a micrographic image of the lung tissue of PBS-administered group (control group) of SPC-IL-18 TG mice in example 3 (HE staining, × 40 magnification).
FIG. 7 shows a micrographic image of the lung tissue of TRX-administered group of SPC-IL-18 TG mice in example 3 (HE staining, × 40 magnification).
FIG. 8 shows the result of immunohistochemical staining of the lung tissue of healthy subjects in reference example 1. The result shows the level of expression of IL-18 (immunohistochemical staining, × 400 magnification).
FIG. 9 shows the result of immunohistochemical staining of the lung tissue of COPD patients in reference example 1. The result shows the level of expression of IL-18 (immunohistochemical staining, × 40 magnification).
FIG. 10 shows the result of immunohistochemical staining of the lung tissue of COPD patients in reference example 1. The result shows the level of expression of IL-18 (immunohistochemical staining, × 200 magnification).
FIG. 11 shows the result of immunohistochemical staining of the lung tissue of healthy subjects in reference example 2. The result shows the level of expression of TRX (immunohistochemical staining, × 40 magnification).
FIG. 12 shows the result of immunohistochemical staining of the lung tissue of healthy subjects in reference example 2. The result shows the level of expression of TRX (immunohistochemical staining, × 200 magnification).
FIG. 13 shows the result of immunohistochemical staining of the lung tissue of COPD patients in reference example 2. The result shows the level of expression of TRX (immunohistochemical staining, × 40 magnification).
FIG. 14 shows the result of immunohistochemical staining of the lung tissue of COPD patients in reference example 2. The result shows the level of expression of TRX (immunohistochemical staining, × 200 magnification).
FIG. 15 shows DNA sequence of mature IL-18cDNA with signal peptides.
FIG. 16 shows recombinant genes SPC-IL-18SP used in the present invention.

### DESCRIPTION OF THE PREFERRED EMBODIMENTS

### Protease

Protease is an enzyme that cleaves protein to their component peptides. Protease includes, for example, metalloprotease, cysteine protease, serine protease, aspartic acid protease (acid protease).

Metalloprotease is a protease that has in its active center heavy metal such as zinc. Metalloprotease includes, for example, matrix metalloprotease (referred to below as MMP), thermolysin, and the like.

MMP is a zinc-containing protease that cleaves adhesive matrix protein between cells. MMP plays a roll in cell division and morphogenesis, as well as cancer metastasis. About 30 kinds of MMP have been identified as MMP-1, MMP-2, - - - , MMP-28.

Cysteineprotease is a protease that has in its active center a cysteine residue. Cysteineprotease includes, for example, caspase, papain, and the like. Among about 20 kinds of caspase (e.g. caspase-1, caspase-2, caspase 3, · · ·), caspase-1, caspase-3, and caspase-9 are important targets of the protease inhibitors of the present invention.

Caspase cuts C-terminal side of aspartic acid. Caspase exists as an inactive precursor before it is cut by an apoptotic signal to be in active form. For example, Caspase-1 (also called as interleukin1 β -converting-enzyme inhibitor) cuts IL-18 precursor to convert it into active IL-18.

Serinprotease includes, for example, elastase and other proteases such as chymotrypsin, subtilisin, and the like.

Elastase is known as a protease that hydrolyzes elastin, which is a main component of elastic fiber of the lung.

Aspartic acid protease includes, for example, pepsin, cathepsin D, and the like.

The protease inhibitors and the preventive and therapeutic agents of the present invention for COPD, AIDS, pulmonary alveolar proteinosis, cardiac failure, hepatic insufficiency, and cardiovascular diseases (e.g. circulatory failure accompanied by pulmonary hypertension)

### Protein with redox activity

The protease inhibitors and the preventive or therapeutic agents for COPD or AIDS of the present invention include (1) to (4) below, solely or in combination, as its active ingredient.
(1) redox activity protein
(2) protein with a similar activity to the redox activity protein, comprising an amino acid sequence in which one or several amino acids are deleted from, replaced with, or added to the redox activity protein
(3) genes that encode (1)
(4) genes that encode (2)

The equivalent activity to that of protein with redox activity means redox regulation activity described below.

Protein with redox activity, having both reduction and oxidation (redox) activity, is capable of redox regulation (regulation of reduction and oxidation). The protein with redox activity refers to peptides with redox activity as well. The protein with redox activity includes, for example, polypeptides of the thioredoxin family, HO-1 (heme oxygenase-1), and the like.

The polypeptide of the thioredoxin family (referred to below as "TRX") refers to polypeptide that has redox activity with respect to a disulfide bond and from a didanosine dithiol bond. The polypeptide of the thioredoxin family (referred to below as "TRX-P" is a polypeptide that originally exists in cells.

The term "TRX" as used herein refers to natural polypeptides that are extracted from animals (including human beings), plants, Escherichia coli, yeasts, and the like. The term "TRX" also refers to polypeptides that are extracted from yeasts, Escherichia coli, and the like using DNA recombination method, as well as polypeptides that are chemically synthesized. Among the polypeptides above, polypeptide derived from human beings, polypeptides prepared by using transgenesis, and synthesized polypeptides of an equivalent or similar sequence are preferable because they have less undesirable effect on subjects than other polypeptides mentioned above.

TRX-P has an active site (-Cys-X1-X2-Cys-: X1, X2 are the same or different amino residues) including a cysteine residue. TRX-P includes a group of molecules having a similar three-dimensional structure.
Therefore TRX-P of the present invention further includes polypeptides where a part of amino acid sequence is deleted or substituted and polypeptides combined with other amino acids or peptides.

The active site of TRX-P can be, for example, -Cys-Gly-Pro-Cys-, -Cys-Pro-Tyr-Cys-, -Cys-Pro-His-Cys-, -Cys-Pro-Pro-Cys-, and the like. Among these, -Cys-Gly-Pro-Cys- is preferable because it is common in various species. Using the polypeptide with such an active site, the results in experiments using mouse models can be more reliably applicable to human beings.

"TRX-P" includes, for example, thioredoxin with an active site of -Cys-Gly-Pro-Cys-, glutaredoxin with an active site of -Cys-Gly-Pro-Cys-, and the like.

TRX can be derived from human-beings, Escherichia coli, and yeasts. Glutaredoxin can be derived from human-beings and Escherichia coli.

As methods of extracting TRX-P from cells of human-beings, the methods below can be exemplified:
(A) Extract TRX-P from cell strain derived from human-beings
   (See Japanese publication of unexamined patent application Tokukai H1-85097)
(B) Use transgenesis method
   (See Japanese publication of unexamined patent application Tokukai H1-85097)
(C) Use peptide synthesis
   (See Japanese publication of unexamined patent application Tokukai H5-139992)

### IL-18 Inhibitors

The preventive or therapeutic agents for COPD, pulmonary alveolar proteinosis, and cardiovascular diseases of the present invention include, (1) to (4) below, solely or in combination, as its active ingredient.
(5) interleukin-18 inhibitor
(6) protein with an activity of inhibiting interleukin-18, comprising an amino acid sequence in which one or several amino acids are deleted from, replaced with, or added to the interleukin-18 inhibitor
(7) genes that encode (1)
(8) genes that encode (2)

IL-18 inhibitors of the present invention can be a substance that inhibits conversion of IL-18 precursor to active IL-18, a substance that neutralize the activity of IL-18 (e.g. IL-18 binding protein and anti-IL-18 antibody), a substance that inhibits binding of IL-18 to an IL-18 receptor, such as recombinant (The Combination of soluble IL- 18Rα and IL- 18Rβ Chains Inhibits IL-18-induced IFN-γ (journal of Interferon and cytokine research 22:P.593-601, 2002, Mary and Liebert, Inc.)) soluble IL-18 receptor or natural soluble IL-18 receptor), a substance that inhibits signal transduction after IL-18 binds to an IL-18 receptor, or the gene encoding those substances.

Many compounds are known as IL- 1 β converting enzyme inhibitors. These compounds include polypeptides having a similar sequence to that of IL- 1 β precursor at a site having affinity for ICE. For example, peptides having a peptide sequence of Tyr-Val-Ala-Asp are known to inhibit the binding of ICE to an IL-1*β* precursor (See paragraph 2 and Description of the Related Art in Japanese publication of unexamined patent application Tokukai H11-147895). The four peptide sequences are the same as the peptide sequence of IL- 1β from the cleavage site (Asp116) to N-terminal side. Examples of the peptides are the peptide derivative disclosed in Japanese publication of unexamined patent application Tokukai H5-255218, the sulfonamide derivative disclosed in Japanese publication of unexamined patent application Tokukai H11-147873, the peptide derivative disclosed in Japanese publication of unexamined patent application Tokuhyo H10-504285, the Glycin derivative disclosed in Japanese publication of unexamined patent application Tokukai H11-147895, tetrazole derivative disclosed in international publication WO97/24339, and the like.

IL-18 binding protein refers to protein disclosed in Immunity, 10, 127-136(1999) and its subclasses. Specifically, IL-18 refers to protein encoded by the gene shown at the bottom of p. 136 in the text as Gen Bank accession number AF 110798 or the subclasses of the protein. The subclasses include protein encoded by the gene shown as GenBank accession number AF11079, AF110800, AF110801, AF110802, AF100803, AF100460, and the like. The protein and its subclasses can be prepared by using the method described in Immunity, 10, 127-136 (1999).

Monoclonal antibodies specific for IL-18 can be prepared by using the method described in J.Immunol.Methods, 217,97-102(1998).

The examples of the substance that inhibits binding of IL-18 to an IL-18 receptor are, for example, IL-18 receptor protein, monoclonal antibodies specific for IL-18 receptors, and the like. The monoclonal antibodies specific for IL-18 receptors include, for example, H44 monoclonal antibody (an antibody against Human IL-18R (α chain)) (See Kitasato. Y., Hoshino, T., Okamoto, M., Kato, S., Koda, Y., Nagata, N., Kinoshita, M., Koga, H., Yoon, D. Y, Asao, H., Ohmoto, H., Koga, T., Rikimaru, T., and Aizawa, H. Enhanced expression of interleukin-18 and its receptor in idiopathic pulmonary fibrosis. Am J Respir Cell Mol Biol, 31:619-625, 2004.)

The monoclonal antibodies specific for IL-18 receptor described above can be antibodies derived from mammals, chimera antibody, or humanized antibody.

The monoclonal antibodies specific for IL-18 receptor protein and for IL-18 receptor can be prepared by using, for example, the method described in Japanese publication of unexamined patent application Tokukai H11-100400.

The substances that inhibit signal transduction after IL-18 bind to an IL-18 receptor can be, for example, those that inhibit IL-18 signal transduction molecules, such as Myd88, IRAK (IL-1receptor-associated kinase), TRAF 6 (TNF receptor-associated Factor), TAK-1 (TGF-β activated kinase), MAPKK3,4,6 (MAP kinase kinase), JNK (c-Jun N-terminal kinase), p38, NIK (NF- κ B-inducing kinase, IKK (Iκ B-kinase), and the like. Such substances include, for example, p38MAP kinase inhibitors (e.g. SB203580, SB220025, RWJ 67657 (American Journal of Respiratory and Critical Care Medicine vol. 160 pp S72-S79, 1999), NF- κ inhibitors (e.g. I- κ B inhibitor, I- α B α gene transfer (American Journal of Respiratory and Critical Care Medicine vol. 160pp S72-S79, 1999) and PS-341 (Proc.Natl.Acad.Sci.USA vol. 95 PP. 15671-15676, December 1998 Medical Sciences), and the like.

The IL-18 inhibitors of the present invention include the IL-18 inhibitors described above and, if the IL-18 inhibitor is a polypeptide, the gene encoding the polypeptide, protein having an amino acid sequence where one or several amino acids are deleted, substituted, or added with respect to the amino acid sequence of the IL-18 inhibitor, or the gene encoding them.

The preventive or therapeutic agents of the present invention may include as its active ingredient a combination of the substances (1) to (4) that are related to the protein with redox activity described above and the substances (5) to (8) that are related to the IL-18 inhibitors.

The content of the active ingredient in the protease inhibitors or the preventive or therapeutic agents for diseases cannot be limited to a certain range but may vary according to the dosage form. The content may be defined within the range that allows the agents to have a desired drug form according to the dosage. For example, the content in solution can be 0.0001 to 10 (w/v%) and preferably be 0.001 to 5 (w/v%). The content in injection can be 0.0002 to 0.2 (w/v%) and preferably be 0.001 to 0.1 (w/v%). The content in solid drug can be 0.01 to 50 (w/w%) and preferably 0.02 to 20 (w/w%). These contents are not necessarily limited within the ranges.

The dosage of the protease inhibitors and the preventive or therapeutic agents of the present invention may vary according to the route of administration, symptom, patient's age, body weight, and the drug form of the preventive or therapeutic agents. The dosage of the protease inhibitors and the preventive or therapeutic agents of the present invention is selected so that the content of their active ingredients may range 0.005 to 50 mg and preferably 0.1 to 100 mg per 1 kg of the subject's body weight. Adult dosage of the protease inhibitors and the preventive or therapeutic agents of the present invention is 0.01 mg at minimum (preferably 0.1 mg) and 20 g at maximum (preferably 200 mg, more preferably 500 mg, most preferably 100 mg). The agents of that dosage may be administered at one time per day or in several doses per day according to the seriousness of the disease.

The protease inhibitors and the preventive or therapeutic agents of the present invention may be combined with conventional preventive or therapeutic components for the same target diseases as those of the present invention. The conventional preventive or therapeutic components include, for example, (1) to (5) below and the like.
(1) Mediator Antagonists:
   LTB4 antagonists (e.g. LY29311, SC-53228, CP-105, 696, SB201146, BIIL284), 5'-Lipxygenase inhibitors (e.g. zileutin, Bayx1005), chemokine inhibitors, IL-8 antagonists (e.g. SB225002; CXCR2 antagonists), TNF inhibitors (e.g. monoclonal Ab, soluble receptors, MMPinhibitors), antioxydants (e.g. NAC, NAL, glutathione, superoxide dismutase, and the like), prostanoid inhibitors (e.g. COX-2 inhibitors, thromboxane antagonists, isoprostane receptor antagonists), iNOS inhibitor, and the like are the examples of mediator antagonists.
(2) Anti-inflammatory Drugs:
   Phosphodiesterase 4 inhibitors (e.g. SB207499, CP80633, CDP-840), adhision inhibitors (e.g. anti-CD11/CD18, anti-ICAM1, E-selectin inhibitors), prostaglandin E analogs (e.g. misoprostil, butaprost), cytokines (e.g. IL-10), colchicine, macrolide antibiotics (e.g. erythromycin, clarithromycin, roxithromycin) and the like are the examples of anti-inflammatory drugs.
(3) Protease Inhibitors:
   For example, neutrophil elastase inhibitors (e.g. ICI200355, ONO-5046, MR-889, L658,758), Cathepsin inhibitors (e.g. suramin), matrix metalloprotease inhibitors (e.g. batimastat, marimastat, KBR 7785), alphal-antitrypsin (e.g. purified, human recombinant, gene transfer), secretory leukoprotease inhibitor, elafin, and the like are the examples of protease inhibitors.
(4) Immunoregulators:
   For example, immunosuppressive agent FK506 and the like are the examples of immunoregulators.
(5) Therapeutic agents for inflammatory respiratory diseases and for respiratory hypersensitivity:
   Xanthine derivatives (e.g. theophylline), β2 receptor stimulating agent, anticholinergic agent, antiallergenic drug, steroids (e.g. adrenocortical hormone drug and steroid inhalant) and the like are the examples of therapeutic agents for inflammatory respiratory diseases and for respiratory hypersensitivity.

The protease inhibitors and the preventive or therapeutic agents for diseases may contain other components as long as their inhibitory effect, or preventive or therapeutic effect is maintained. The protease inhibitors and the preventive or therapeutic agents for diseases may be formulated with pharmaceutically acceptable carriers such as excipients, lubricants, binders, disintegrators, stabilizers, flavoring agents, diluents, surfactants, emulsifiers, solubilizers, absorption promoters, moist retainers, adsorbents, fillers, volume expanders, moisteners, antiseptics, and other additives.

The excipients can be organic excipients, inorganic excipients, and the like.

The protease inhibitors and the preventive or therapeutic agents of the present invention are mainly for oral administration. The protease inhibitors and the preventive or therapeutic agents of the present invention are administered orally in the forms of, for example, tablets, capsules, granules, powders, pills, troches, syrups, or the like.

Alternatively the protease inhibitors and the preventive or therapeutic agents of the present invention for the diseases may be administered non-orally by, for example, intravenous administration (e.g. intravenous injection), intramuscular injection, transdermal administration, intradermal administration, subdermal administration, intraperitoneal injection, intrarectal administration, mucosal administration, inhalation, and the like. Intravenous administration (e.g. intravenous injection) is the most preferable in terms of safety and for a stable blood level of the protease inhibitors agents of the present invention.

The gene encoding the protein with redox activity described above can be used as a protease inhibitor or as a preventive or therapeutic agent for COPD or for AIDS in a gene therapy. If the IL-18 inhibitor is a polypeptide, the gene encoding the IL-18 inhibitor can be used as a preventive or therapeutic agent for COPD, pulmonary alveolar proteinosis, and cardiovascular diseases in a gene therapy.

The gene can be used in the form of DNA, as well as RNA, plasmid, virus vector, and the like. For each form, both single-stranded and double-stranded form can be used.

The plasmid can be used by injecting expressed plasmid by intramuscular injection (DNA vaccination) or by liposome method, Lipofectin method, microinjection method, calcium phosphate method, electroporation method, and the like. Particulary DNA vaccination and liposome method are preferable.

When virus vectors are used, a desired gene is embedded in a virus.

Viruses that are used in the virus vector can be, for example, DNA viruses and RNA viruses, such as, retrovirus, adenovirus, adeno-associated virus, herpesvirus, vaccinia virus, poxvirus, poliovirus, sindbis virus, and the like. Among these viruses, retrovirus, adenovirus, adeno-associated virus, vaccinia virus, and the like are preferable to be used. Adenovirus is particularly preferable to be used.

To use genes as a medicine, the genes can be introduced directly in the subject's body according to "in vivo method". Alternatively, the genes are introduced in cells taken from human beings and then returned to the subject's body according to "ex vivo method". "In vivo method" is preferable to be used in the present invention.

It is possible to select an appropriate route of administration depending on the disease to be treated and on the seriousness of the disease. The gene can be administered, for example, through vein or artery, or subcutaneously, intradermally, or intramuscularly.

When the genes are administered by "in vivo method", they can be used in the form of, for example, solution. Generally, injectable solution comprising genes as an active ingredient is preferably used. Common carriers can be added to such injectable solution.

Liposome or fusogenic liposome (e.g. Sendai virus (HVJ)-liposome) comprising genes can be used in the form of liposome formulation. Liposome formulation can be, for example, suspensions, cryogen agents, centrifugation-concentrated cryogen agents, and the like.

### Method of inhibiting protease and method of preventing and treating COPD, AIDS, pulmonary alveolar proteinosis, and cardiovascular diseases using the inhibitors and the preventive or therapeutic agents of the present invention

The protease inhibitors and the preventive or therapeutic agents of the present invention for COPD, AIDS, pulmonary alveolar proteinosis, and cardiovascular diseases can be used in the above-described forms for preventing or treating such diseases. Alternatively, the genes that act as the protease inhibitors or the preventive or therapeutic agents of the present invention for COPD, AIDS, pulmonary alveolar proteinosis, and cardiovascular diseases can be used for preventing or treating such diseases.

### Method of preparing disease animal models to be used to verify the effects of the present invention

Pulmonary emphysema animal models for verifying the COPD-inhibiting effect of the present invention can be prepared by using the method described in Shapiro, S. S. animal models for COPD, Chest, 117:223S-227S, 2000. Specifically, pig elastase suspended in a clean PBS can be intracheally administred

### New disease animal models

The present inventor has developed a new COPD animal model (See Japanese publication of unexamined patent application Tokugan 2004-069835 by Hoshino). The new COPD animal models can be used as disease animal models for verifying the COPD-inhibiting effect of the present invention. The new animal models can also be used as animal models of pulmonary alveolar proteinosis and cardiovascular diseases. For the simplicity of the description, the new animal models are referred to below as COPD animal models. The method of preparing COPD animal models is described below.

The COPD animal models used in the present invention are animal models in which recombinant genes comprising any of the genes of (X1) to (Y2) are introduced.
(X1) Interleukin-18 genes
(X2) Genes with a similar activity to the interleukin-18 genes, comprising an amino acid sequence in which one or several amino acids are deleted from, replaced with, or added to the interleukin-18 genes
(Y1) Caspase-1 genes
(Y2) Genes with a similar activity to the caspase-1 genes, comprising an amino acid sequence in which one or several amino acids are deleted from, replaced with, or added to the casepase-1 genes

It has been confirmed that the new COPD animal models are affected by chronic obstructive pulmonary diseases (e.g. COPD, pulmonary emphysema, and the like), pulmonary diseases (e.g. pulmonary alveolar proteinosis and the like), circulatory failures (e.g. hepatic insufficiency, cardiac failure such as cor pulmonale and pulmonary hypertension), and the like within 5 to 8 weeks after birth. Conventionally, COPD animal models were prepared by repeatedly administering tobacco as a substance causing inflammation for a long period as long as six months. The new COPD animal models can be prepared in comparatively shorter period and in a simpler manner without administering substances causing inflammation such as pig elastase and papain.

### Recombinant genes

Recombinant genes that are introduced in the animal models can be obtained by putting the genes (X1) or (X2) below (referred to below as "IL-18 genes" collectively) or (Y1) or (Y2) below (referred to below as "caspase genes" collectively) under promoters expressed specifically in the lung.
The promoters expressed specifically in the lung can be, for example, promoters derived from lung cells (e.g. lung surfactant promoter, clara cell promoter, or the like).
(X1) IL-18 genes
(X2) Genes with a similar activity to the IL-18 genes, comprising an amino acid sequence in which one or several amino acids are deleted from, replaced with, or added to the IL-18 genes
(Y1) Caspase-1 genes
(Y2) Genes with a similar activity to the caspase-1 genes, comprising an amino acid sequence in which one or several amino acids are deleted from, replaced with, or added to the casepase-1 genes

The activity that is equivalent to that of IL-18 refers to signal transduction through IL-18 receptor and the like. The examples of such activity include interferon γ (IFN-γ) inducing activity. The activity that is equivalent to that of caspase-1 refers to an activity for cutting IL-18 precursor to be active IL-18 (mature IL-18).

The lung surfactant promoter can be, for example, human lung surfactant promoter (surfactant protein-C gene promoter; referred to as "SPC promoter" hereinafter) and the like. SPC promoter can be obtained, for example, according to the method described in "Early restriction of peripheral and proximal cell lineages during formation of the lung" (Proc Natl Acad Sci USA. 2002 Aug 6;99(16):10482-7).

The clara cell promoter can be, for example, CC10 promoter (sometimes called CCSP) and the like. CC10 promoter can be obtained, for example, according to the method described in "cis-acting elements that confer lung epithelial cell expression of the CC10 gene" (J Biol Chem. 1992 Jul 25;267(21):14703-12.)

These promoters can be used to efficiently prepare disease animal models of the present invention.

The recombinant genes preferably comprises, for example, signal peptide (SP) genes that facilitate emission of the introduced genes outside the cells, Kozak sequence that optimizes protein expression, and poly(A) sequence that is helpful to, for example, pick up the expressed genes.

The signal peptide is an amino acid sequence with enough hydrophobicity to pass through cell membranes that are composed of lipids while the genes are secreted outside the cells. After passing through the membrane, the signal peptide is cut by an enzyme (signal peptidase).

The signal peptide can be, for example, mouse immunoglobulin (referred to as "Ig" hereinafter) κ -chain signal peptide and the like. Mouse immunoglobulin (referred to as "Ig" hereinafter) κ -chain signal peptide is described in, for example, "Hybridoma fusion cell lines contain an aberrant kappa transcript" (Carroll, W.L., E. Mendel, S. Levy. 1985. Mol. Immunol. 25:991).

The Kozak sequence is a bacterium-derived DNA sequence that is found near ATG start codon of genes. The Kozak sequence comprises comparatively much guanine and cytosine. The Kozak sequence helps optimizing protein expression and is usually used for cloning (See, for example, Nucleic Acids Res. 1984 Jan 25; 12(2):857-72. Compilation and analysis of sequences upstream from the translational start site in eukaryotic mRNAs).

Poly(A) sequence is a nucleotide sequence comprised of successive adenylate acids (A).

Poly(A) sequence can be, for example, bovine poly(A) sequence, and the like (See, for example, Goldman, L.A., E.C. Cutrone, S.V. Kotenko, C.D. Krause, J.A. Langer. 1996. Modifications of vectors pEF-BOS, pcDNA1 and pcDNA3 result in improved convenience and expression. Bio Techniques 21:1013).

If the IL-18 genes are used to prepare the recombinant genes, known techniques can be used for facilitating emission of the introduced genes outside the cells beside the above-described technique of introducing signal peptide. For example, IL- 1 β converting enzyme (caspase-1) genes for converting proIL-18 to active IL-18 in addition to IL-18 genes can be introduced to animal models to allow both IL- 1 β converting enzyme (caspase-1) genes and IL-18 genes to be expressed.

### Disease animal models

Disease animal models can be prepared by, for example, the methods described below.

Animals such as rodents, dogs, cats, monkeys, horses, pigs, and the like can be used in the present invention. Rodents can be, for example, mice, rats and the like but mice are preferable. Among mice, C57BL/6N mice (also called B6 mice), Balb/c mice, and the like are preferable. B6 mice are most preferable.

Description below will be made taking an example where mice are used as the transgenic animals. However, the present invention is not limited to this example.

Known transgenesis methods can be used in addition to the methods described above. The examples of the methods are shown below. The description below will be made taking an example of IL-18 gene but the same methods can be used for the IL-18 associated genes ((X2) above) and for caspase1 genes ((Y1) and (Y2) above).

Signal peptides taken from V-J2-C site of mouse Ig κ -chain and mouse pro-IL-18cDNA (See reference (1): Hoshino T, Kawase Y, Okamoto M, Yokota K, Yoshino K, Yamamura K, Miyazaki J, Young HA, Oizumi K. Cutting edge: IL-18-transgenic mice: invito evidence of a broad role for IL-18 in modulating immune function. J Immunol 2001;166:7014-7018) are used to obtain mature IL-18 cDNA with signal peptides according to PCR method (See reference (1) and reference (2): Kawase Y, Hoshino T, Yokota K, Kuzuhara A, Kirii Y, Nishiwaki E, Maeda Y, Takeda J, Okamoto M, Kato S, Imaizumi T, Aizawa H, Yoshino K. Exacerbated and Prolonged Allergic and Non-Allergic Inflammatory Cutaneous Reaction in Mice with targeted Interleukin-18 Expression in the Skin. J invest Dermatol 2003;121:502-509). Any Pro-IL-18cDNA that becomes gene (X) or (Y) when it becomes mature IL-18 can be used.

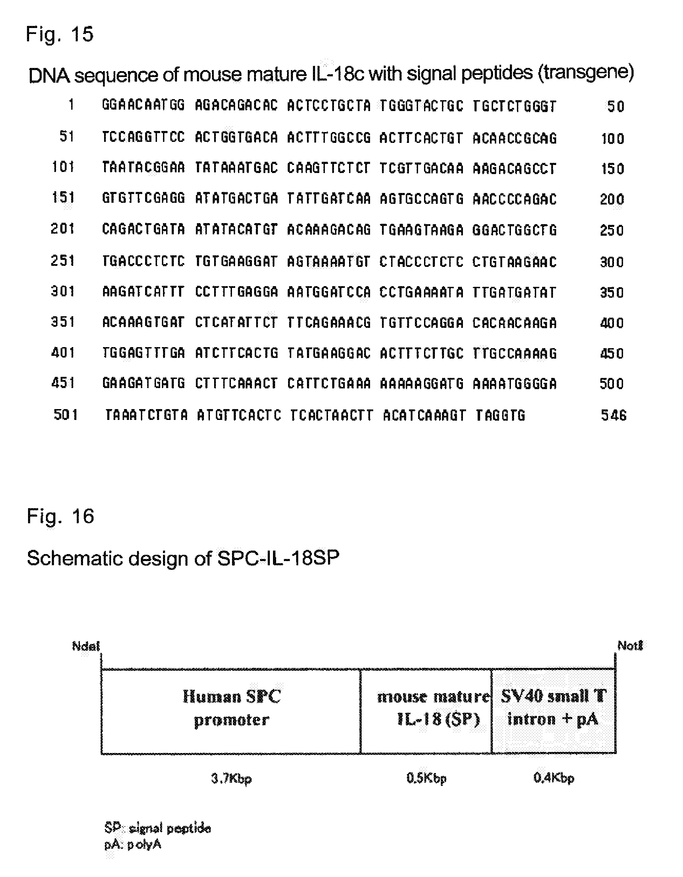

The sequence (DNA sequence) of mature IL-18 cDNA with signal peptide is shown in Fig. 15 and Sequence 1. In the sequence of Fig. 15 (Sequence 1), start codon of 7 to 9 th amino acid is followed by G of 10th amino acid. From the 10th amino acid G to the 69th amino acid C are V-J2-C site-derived signal peptide genes of mouse Ig κ chain. The 69th amino acid C is followed by AAC codon. The AAC codon to "AGT" codon right before "TAG" stop codon are mature IL-18cDNA.

"CGAACA" including Kozak sequence is a sequence that optimizes protein expression. The sequence originally exists in pro-IL-18cDNA genome of mice.

"GTG" after STOP codon originally exists in pro-IL-18cDNA genome of mice but the sequence is not necessary.

Then pCR2.1 vector (available from Invitrogen) is used for cloning PCR products and sequencing (See reference 1 and 2). Then, 3.7SPC/SV40 vector (Proceedings of the National Academy of Sciences of the United States of America, August 6, 2002 vol. 99 no. 16 10482-10487) comprising human surfactant promoter, such as SPC (Early restriction of peripheral and proximal cell lineages during formaiton of the lung. Proc Natl cad Sci USA. 2002 Aug 6;99(16):10482-7.), SV40 small T intron (Early restriction of peripheral and proximal cell lineages during formaiton of the lung. Proc Natl Acad Sci USA. 2002 Aug 6;99(16):10482-7.) and bovine poly(A) (Goldman, L.A., E.C. Cutrone, S.V. Kotenko, C.D. Krause, J.A. Langer. 1996, Modifications of vectors pEF-BOS, pcDNA1 and pcDNA3 result in improved convenience and expression. BioTechniques 21:1013.) is cut with Eco RI (available from New England (MA, USA)). The PCR product is integrated to the Eco RI site for subcloning to obtain SPC-IL-18SP (Fig. 16).

SPC-IL-18SP is cut by restriction enzymes, NdeI (New England Biolabs (MA, USA)) and Notl (New England Biolabs (MA, USA)) at 37 °C for two or more hours (according to the protocol by New England Biolabs (MA, USA)) to obtain linear DNA fragments.

Recombinant genes can be introduced to mice by known transgenic methods. For example, the recombinant genes (linear DNA fragment) that are obtained according to the above-described method are injected to a fertilized egg of a mouse. Then the egg is inserted in the fallopian tube of a surrogate mother to obtain an SPC-IL-18TG mouse (founder). For verifying that the recombinant genes has been safely injected to the fertilized egg of a mouse, DNA of the child mouse is extracted from its tail using DNA easy kit (available from Qiagen, Germany) to be checked with PCR. The child mouse is mated with a wild male mouse that is non-syngenic with the surrogate mother. From the descendants of the child mouse (both male and female descendants including F2, F3, ....), IL-18 expressed mice are selected to be used as transgenic mice. The selection among the descendants is carried out by PCR analysis on genome DNA from their tail, ELISA analysis on mature IL-18 in blood serum, western blotting analysis on mature IL-18 in the lung, the heart, the lever, and the like (See reference 1 and 2 above).

IL-18 genes that are controlled by a promoter to be expressed specifically in the lung are introduced in a mouse. The amount of the IL-18 genes can be selected depending on the kind of mouse, desired onset timing, and desired seriousness of the disease. Generally 1 ng/lung (50 ng/kg weight) to 10 ng/lung (500 ng/kg weight) of the IL-18 gene is introduced to the lung of mice.

IL-18 gene is introduced to the animal models so that, for example, expressed mature IL-18 in mouse blood serum can be 1 to 10 ng/mL. The more IL-18 gene is introduced, more target diseases (e.g. pulmonary diseases and cardiac diseases exemplified above) are caused. Various diseases can be caused simultaneously in the model mice described above. If a pulmonary disease and a cardiac disease are caused simultaneously, their lesion sites are apart from each other. Therefore it is easy to distinguish for which site the tested agent is effective while screening the agents. If several diseases are caused in one lesion site, it is possible to tell which disease is expected to be cured by the tested agent by analyzing the organ of the lesion site.

SPC-IL-18 is not necessarily expressed in a constant period from the time of its introduction. SPC-IL-18 is expressed in about 4 weeks after birth in some mice and in about 5 weeks after birth in most mice. The older the mouse is, more seriously the diseases occur. At an early stage, each mouse is affected by different diseases of different seriousness. After 5 to 8 weeks after birth, most pulmonary diseases and cardiac diseases mentioned above are caused in most mice.

The disease animal models prepared according to the above-mentioned method can be used for pulmonary diseases (e.g. chronic obstructive pulmonary disease, pulmonary alveolar proteinosis, and the like), circulatory failures (e.g. hepatic insufficiency, cardiac failure such as cor pulmonale, pulmonary hypertension). These animal models can be used for screening the preventive or therapeutic agents for these diseases.

### Example 1

### Test on protease-inhibiting effect of TRX in a test tube

Inhibiting effect of TRX for caspase-1, MMP-1, MMP-9 was tested.

### Assay method:

### Caspase-1

A test according to Thornberry NA (Nature 356(30):768-775, 1992) was carried out. Specifically, recombinant human caspase-1 was allowed to react with 20 µ M Ac-YVAD-AMC at 37 °C for 3 hours. After that, the fluorescent level of AMC (7-amino-4-methylcoumarin) was determined twice (the determination was carried out by MDS Pharma Services Japan (Kyoto, Japan))

### MMP-1,9

Recombinant MMP-1 (peptide laboratory (Kyoto, Japan)), recombinant MMP-9 (peptide laboratory (Kyoto, Japan)) were allowed to react with 50 *µ* M P3163-v (MOCAc-Pro-Leu-Gly + Leu-Azpr(DNP)-Ala-Arg-NH2) (peptide laboratory (Kyoto, Japan)) at 37 °C for 2 hours before the fluorescent level of AMC (7-amino-4-methylcoumarin) was determined twice.

### Results

Under the presence of 100 µ g/mL purified TRX, caspase-1 was suppressed by 21 %. Also, MMP-1 and MMP-9 were suppressed respectively by 47 % and by 76 % under the presence of 100 µ g/mL purified TRX.

This shows that TRX has an inhibitory effect with respect to protease such as cystein protease and metalloprotease.

### Example 2

### Test on COPD-inhibiting effect of TRX using traditional animal models

The effect of therapeutic agents for COPD was tested using the traditional COPD animal models prepared with elastase according to the above-mentioned method. Five 8-week-old C57BL/6N mice were used for each group 1 to 4 below.
(Group 1) 100 µ L clean PBS was intratracheally administered with a syringe on day 1 (control mice)
(Group 2) Pig elastase (produced by SIGMA, 0.3U) suspended in 100 *µ* L clean PBS was intratracheally administered with a syringe on day 1. (comparison 1: pathologic mouse models 1)
(Group 3) Ovalbumin (OVA) (produced by SIGMA, 40 µ g) suspended in 100 µ L clean PBS was intraperitoneally injected every two days from day 0 to day 20. OVA was administered as a control against TRX. Also, pig elastase (produced by SIGMA, catalogue no. E1250, 0.3U) suspended in 100 *µ* L clean PBS was intratracheally administered with a syringe on day 1. (comparison 2: pathologic mouse models 2)
(Group 4) 40 *µ* g of human recombinant TRX suspended in 100 *µ* L clean PBS was intraperitoneally injected every two days from day 0 to day 20.
Also, pig elastase (produced by SIGMA, catalogue no. E1250, 0.3U) suspended in 100 µ L clean PBS was intratracheally administered with a syringe on day 1 (example 2: predisposing factor + therapeutic agents administered mice).

All mice were disposed of on day 21. The lungs of the mice were fixated by reflux by introducing 20 % formalin through bronchus under a pressure of 15 cm H₂O. Paraffin sections of the lung were subjected to HE staining. Micrographic images were taken with a digital camera for microscopes (DXM1200; available from NIKON). ACT-1 (NIKON) and Photoshop (available from Adobe) were used as softwares for analyzing the images. For both lungs of one mouse, HE stained slides of six different sections (sections of the upper lung field, middle lung filed, and lower lung field sampled from the right lung and from the left lung) were prepared. For each slides, images from five different angles were taken. The images were analyzed with ACT-1. Four grids that are spaced apart with an interval of 300 µ m are drawn with Photoshop (Adobe) on each image. The number of grids that a certain pulmonary alveolus intersects is counted. For example, if the pulmonary alveolus intersects three grids, the average length (mean linear intercept: Lm) of pulmonary alveoli is 300 µm/3 = 100 *µ* m. The Lm of each mouse was calculated with 6 sections × 5 angles × 4 grids = 120 grids. The Lm of each group was analyzed by Welch's t test.

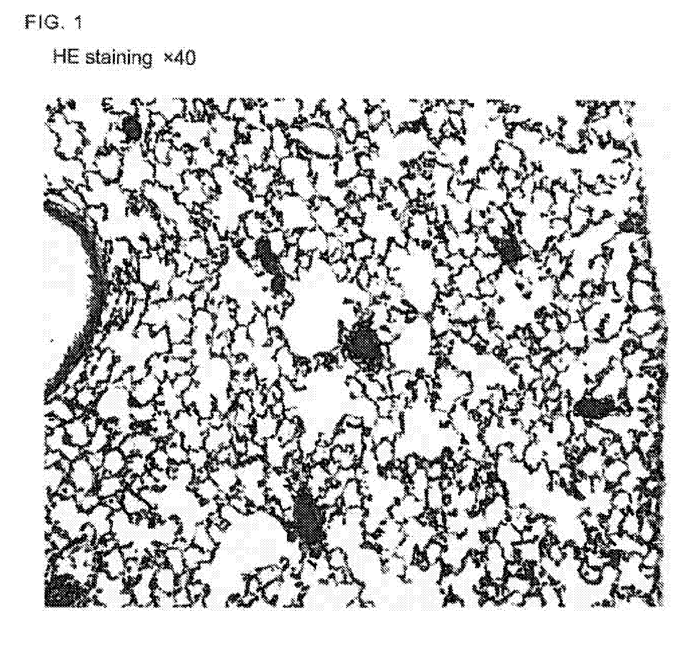

Group 1 did not show a significant histological change (Fig 1).

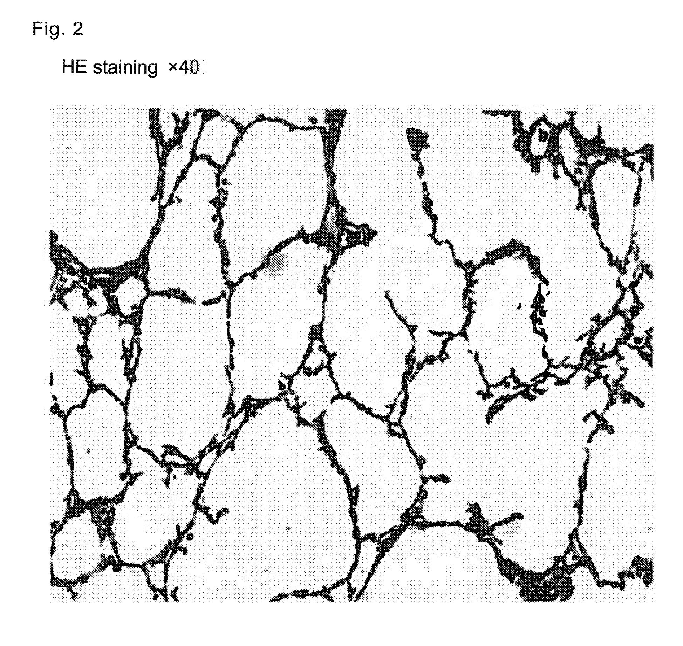

Bronchus lumen of Group 2 was significantly expanded and an experimental pathological COPD occurred (Fig. 2).

Bronchus lumen of Group 3 was significantly expanded and an experimental pathological COPD occurred. The seriousness of COPD was similar to that of Group 2. This shows that OVA, which was administered intraperitoneally as the control protein against redox protein does not inhibit COPD.

No significant changes occurred in the lungs of Group 4. This shows TRX strongly inhibits the experimental pathologic COPD.

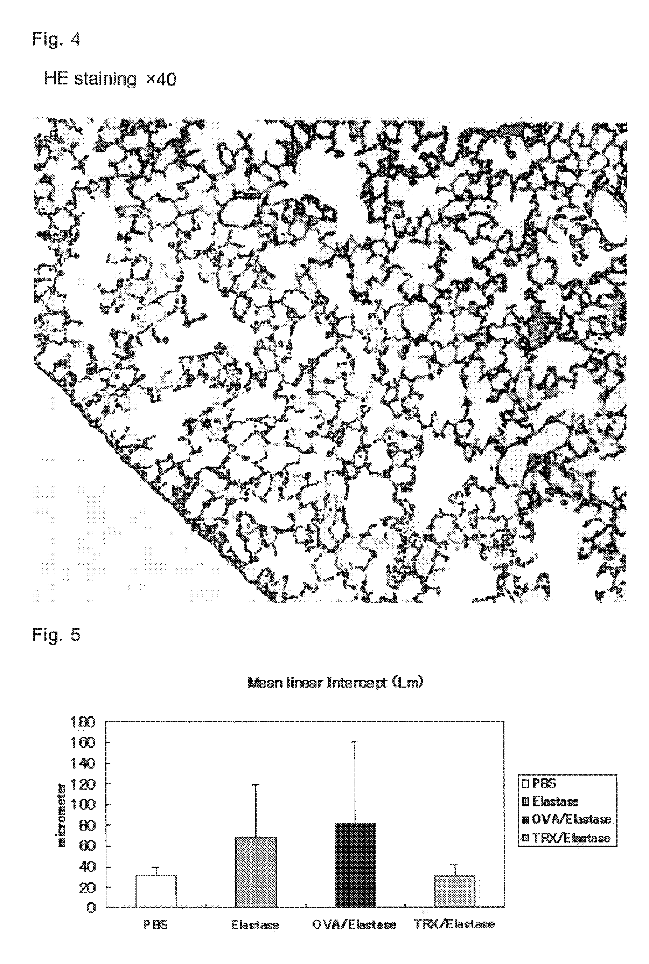

The images of the lungs of each group were examined using the HE slides to calculate Lm of each group. The average Lm of each group was 31.7 for Group 1, 69.0 for Group 2, 82.0 for Group3, 30.4 for Group 4 (Fig 5).

The calculated Lm showed that COPD was more statistically significantly inhibited in Group 4 than in Group 2 (p=4.00x10e-20) and Group 3 (p=4.00x10e-20).

There was no significant difference between Lm of Group 1 and of Group 4.

The results showed that TRX, a kind of protein with redox activity, had a strong and statistically significant inhibitory effect against experimental pathological COPD.

The protein with redox activity inhibited pulmonary emphysema in elastase-induced pulmonary emphysema animal models. This means that the protein with redox activity functions as an elastase inhibitor. Considering also the results of example 1, it is clear that the protein with redox activity or the genes encoding the protein have an inhibitory effect with respect to proteases (e.g. elastase as well as serine protease, metalloprotease, cystein protease, and the like).

### Example 3

### Test on Inhibitory effect of TRX on experimental pathological COPD using new animal models

New animal models described above were used to test the effect of COPD therapeutic agents.

Seven to eight-week-old SPC-IL-18TG mice were prepared according to the above-mentioned method (5 mice for each group). To each group, 400 µ g/mL (40 µ g/mL/mouse) recombinant TRX solved in 0.1 or 0.2 mL sterile phosphate-buffered solution (PBS) (control) was intraperitoneally administered. After 21 days, the lungs of the mice were collected and the lung tissues were HE stained.

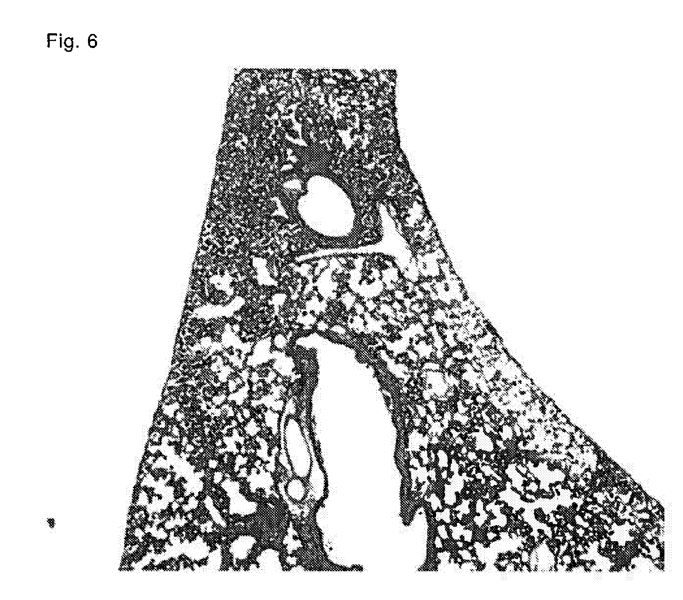

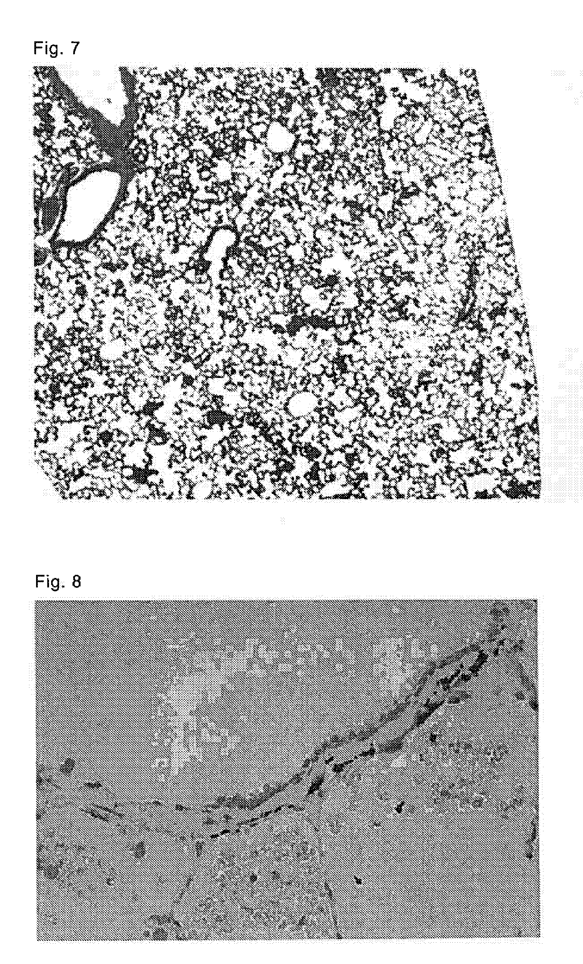

Fig. 6 and Fig. 7 show the results. Experimental pathological COPD was caused in PBS administered controls (Fig. 6) while either COPD or pulmonary alveolar proteinosis were not caused in TRX administered group (Fig. 7). Also, it was verified with HE staining that there are no thickening of pulmonary artery or no marked congestion in the lung. Therefore, it can be concluded that in the TRX-administered mice, cardio vascular diseases (e.g. cardiac failure) was improved in the hearts of the mice.

In the mouse models described above, diseases were caused by IL-18 that is expressed specifically in the lung. TRX is known to have an IL-18 inhibitory effect. Thus, it is clear that the diseases (e.g. COPD) that are caused in the above-mentioned mouse models can be prevented or treated by inhibiting IL-18 signals.

Therefore, the IL-18 inhibitory agents (e.g. anti-IL-18 antibody) can be used as therapeutic agents for COPD, pulmonary alveolar proteinosis, cardiac failure, hepatic insufficiency, cardiovascular diseases (e.g. circulatory failure accompanied by pulmonary hypertension), and the like.

### Reference Example 1

### Strong expression of IL-18 in the lesion site of COPD patients

Paraffin sections of lung tissues from ten COPD patients and from other six people including those who died in traffic accidents were prepared by using formalin fixation. The sections underwent immunohistologic staining with anti-human IL-18 antibody (clone8).

Fig 8 to 10 show the results. IL-18 was not expressed in the lungs of healthy subjects (Fig. 8) as reported in Kitasato, Y, Hoshino, T., Okamoto, M., Kato, S., Koda, Y, Nagata, N., Kinoshita, M., Koga, H., Yoon, D. Y, Asao, H., Ohmoto, H., Koga, T., Rikimaru, T., and Aizawa, H., Enhanced expression of interleukin-18 and its receptor in idiopathic pulmonary fibrosis. Am J Respir Cell Mol Biol, 31:619-625, 2004.

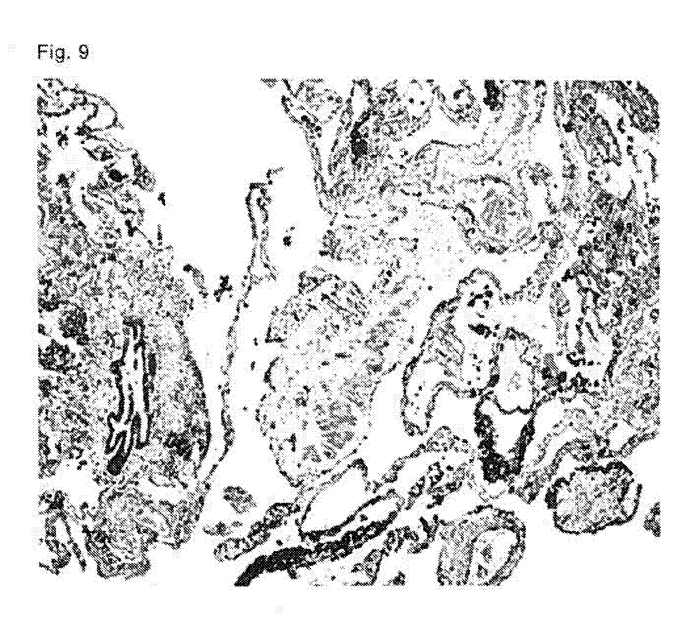

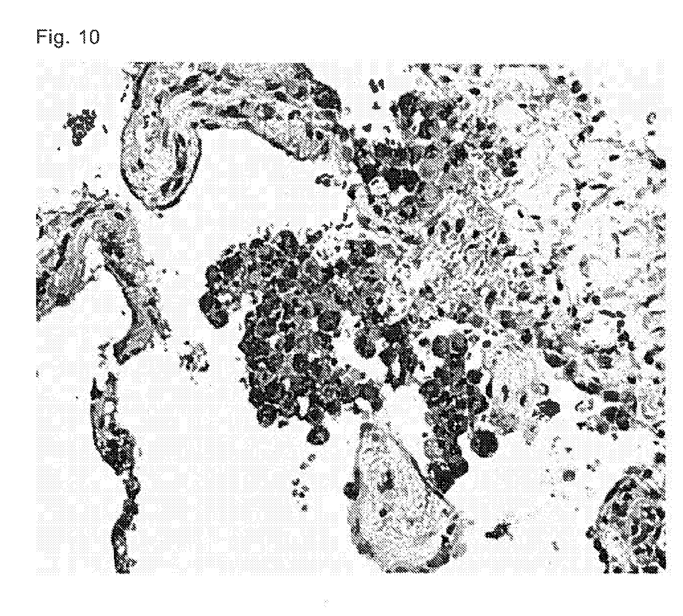

On the other hand, IL-18 was strongly expressed in the lung lesion site of COPD patients. Particularly strong expression of IL-18 was observed in invasive inflammatory cells and in alveolar epithelium (Fig. 9 and Fig. 10). This supports excessive expression of IL-18 in the lung as a cause of COPD.

### Reference Example 2

### Strong expression of IL-18 in the lesion site of COPD patients

Paraffin sections of lung tissues from ten COPD patients and form other six people including those who died in traffic accidents were prepared by using formalin fixation. The sections underwent immunohistologic staining with anti-human IL-18 antibody (produced by Serotec) according to the method described in Kitasato, Y, Hoshino, T., Okamoto, M., Kato, S., Koda, Y, Nagata, N., Kinoshita, M., Koga, H., Yoon, D. Y, Asao, H., Ohmoto, H., Koga, T., Rikimaru, T., and Aizawa, H., Enhanced expression of interleukin-18 and its receptor in idiopathic pulmonary fibrosis. Am J Respir Cell Mol Biol, 31:619-625, 2004.

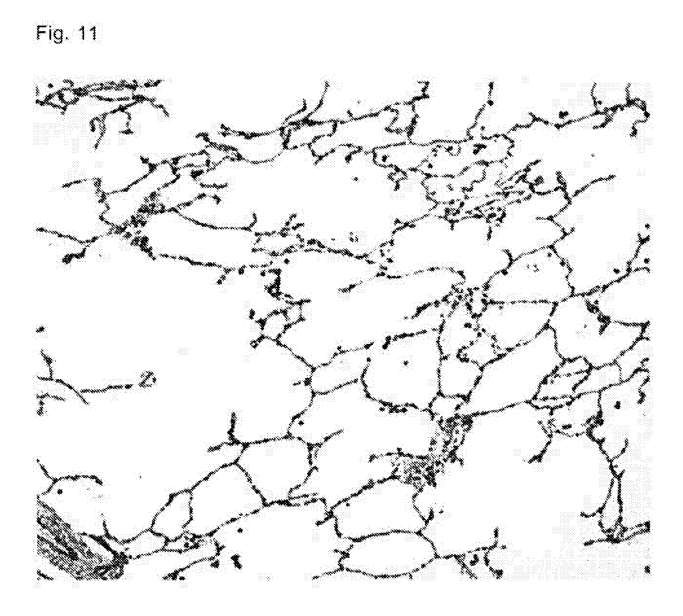

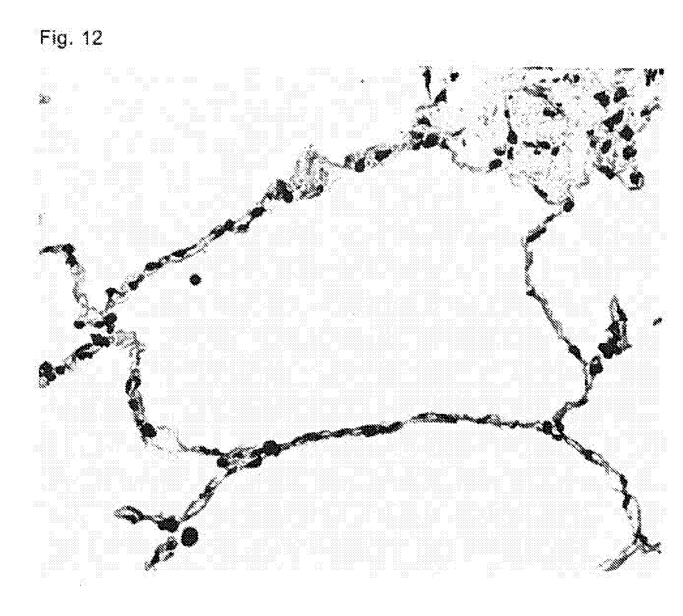

Fig. 11 to 14 show the results. TRX was not expressed strongly in alveolar epithelium of healthy subjects (Fig. 11 and 12).

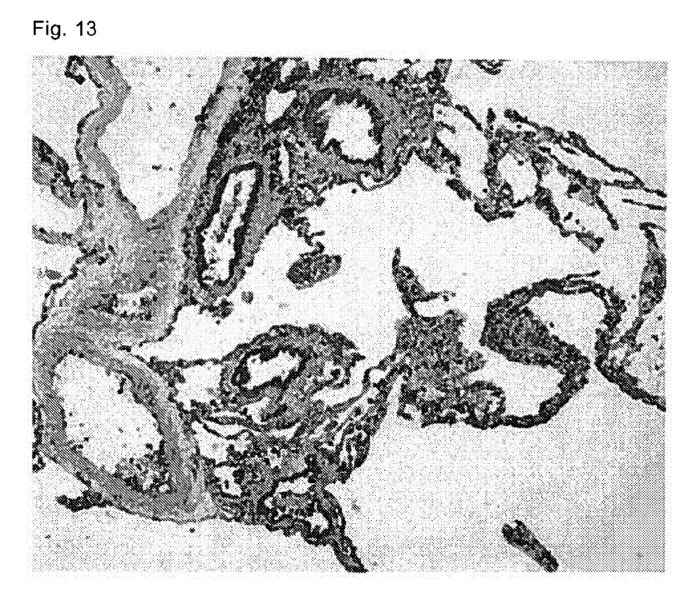

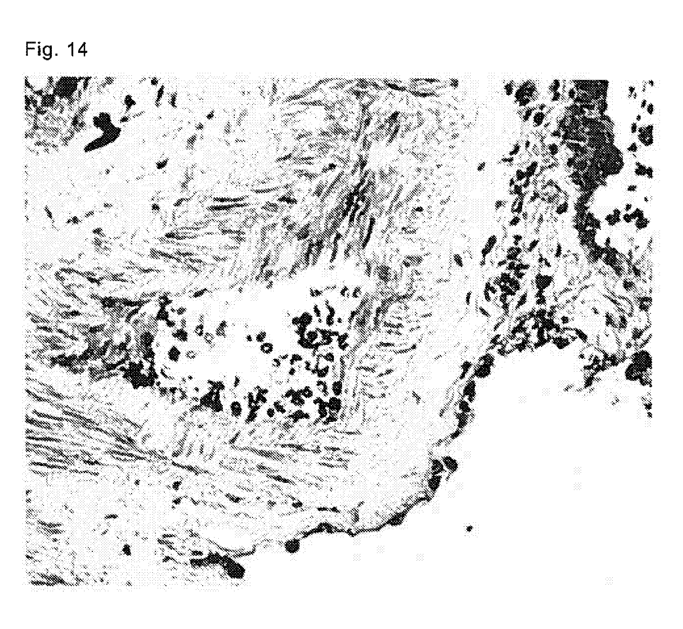

On the other hand, TRX was strongly expressed in the lung lesion site of COPD patients. Particularly strong expression of IL-18 was observed in invasive inflammatory cells, alveolar epithelium, fibrolast in bronchus (Fig. 13 and14).

The results of the reference example 1 and 2 support the possibility that TRX is expressed in vivo to inhibit excessive expression of IL-18 in COPD. Therefore the above-mentioned IL-18 inhibitors can function as preventive or therapeutic agents for COPD.

The protease inhibitors of the present invention comprising protein with redox activity or the genes encoding the protein and the preventive or therapeutic agents for COPD strongly inhibit COPD. Also, they can be used in an AIDS therapy as a protease inhibitor used solely or in combination with other drugs for a cocktail therapy (e.g. HAART therapy). The preventive or therapeutic agents of the present invention comprising IL-18 inhibitors or the genes encoding the IL-18 inhibitors can effectively cure COPD, pulmonary alveolar proteinosis, cardiac failure, hepatic insufficiency, cardiovascular diseases (e.g. circulatory failure accompanied by pulmonary hypertension).

## Claims

1. A protease inhibitor comprising at least one of the substances selected from (1) to (4).
(1) redox activity protein
(2) protein with a similar activity to said redox activity protein, comprising an amino acid sequence in which one or several amino acids are deleted from, replaced with, or added to said redox activity protein
(3) genes that encode (1)
(4) genes that encode (2)

2. The protease inhibitor of claim 1,
wherein said protease is selected from a group consisting of metalloprotease, serine protease, and cysteine protease.

3. A preventive or therapeutic agent for chronic obstructive pulmonary disease or for immunodeficiency syndrome comprising at least one of the substances selected from (1) to (4).
(1) redox activity protein
(2) protein with a similar activity to said redox activity protein, comprising an amino acid sequence in which one or several amino acids are deleted from, replaced with, or added to said redox activity protein
(3) genes that encode (1)
(4) genes that encode (2)

4. A preventive or therapeutic agent for chronic obstructive pulmonary disease comprising at least one of the substances selected from (5) to (8).
(5) interleukin-18 inhibitor
(6) protein with an activity of inhibiting interleukin-18, comprising an amino acid sequence in which one or several amino acids are deleted from, replaced with, or added to said interleukin-18 inhibitor
(7) genes that encode (1)
(8) genes that encode (2)

5. A preventive or therapeutic agent for pulmonary alveolar proteinosis comprising at least one of the substances selected from (5) to (8).
(5) interleukin-18 inhibitor
(6) protein with an activity of inhibiting interleukin-18, comprising an amino acid sequence in which one or several amino acids are deleted from, replaced with, or added to said interleukin-18 inhibitor
(7) genes that encode (1)
(8) genes that encode (2)

6. A preventive or therapeutic agent for cardiovascular disease comprising at least one of the substances selected from (5) to (8).
(5) interleukin-18 inhibitor
(6) protein with an activity of inhibiting interleukin-18, comprising an amino acid sequence in which one or several amino acids are deleted from, replaced with, or added to said interleukin-18 inhibitor
(7) genes that encode (1)
(8) genes that encode (2)
